# EUROPÄISCHE PATENTSCHRIFT

(11) **EP 2 060 238 B1**
(45) Veröffentlichungstag und Bekanntmachung des Hinweises auf die Patenterteilung: **15.02.2012**
(21) Anmeldenummer: 08019895.5
(22) Anmeldetag: 14.11.2008
(51) Int. Cl.: A61B 18/14

(54) **Koagulationsinstrument**
Coagulation instrument
Instrument de coagulation

(30) Priorität: 15.11.2007 DE 202007016059 U; 08.05.2008 DE 102008022889
(43) Veröffentlichungstag der Anmeldung: 20.05.2009
(73) Patentinhaber: Hensler, Ewald, 88637 Buchheim (DE)
(72) Erfinder: Hensler, Ewald, 88637 Buchheim (DE)
(74) Vertreter: Patentanwälte Westphal, Mussgnug & Partner

(56) Entgegenhaltungen:
- US-A- 4 686 981
- US-A- 6 059 783
- US-A1- 2006 004 356
- US-A1- 2006 161 149
- US-A1- 2007 073 285
- US-A1- 2007 208 341
- US-B1- 6 206 876
- US-B1- 6 592 580

## Beschreibung

Die Erfindung betrifft ein Koagulationsinstrument gemäß dem Oberbegriff des Patentanspruchs 1.

In vielen Bereichen der Chirurgie werden seit langer Zeit Instrumente eingesetzt, die einen Koagulationseffekt ausnutzen. Eine Koagulierung von menschlichem Gewebe findet statt, falls ausreichend elektrischer Strom durch das Gewebe fließt und damit das Gewebe erwärmt. Um unterwünschte Nebeneffekte wie Muskel- und Nervenreizungen auszuschließen, werden üblicherweise hochfrequente Wechselströme mit Frequenzen von mehr als 300 KHz verwendet.

Der Strom kann dabei entweder zwischen einer kleinflächigen Elektrode und einer großflächigen Elektrode fließen, wodurch Gewebe in der Nähe der kleinflächigen Elektrode koaguliert wird, oder es werden zwei kleinflächige Elektroden verwendet, um eine Koagulation von Gewebe in einem eng begrenzten Bereich, nämlich zwischen den beiden Elektroden, zu erreichen. Mit dieser Methode können beispielsweise Blutgefäße schnell und zuverlässig verschlossen werden.

Problematisch bei der Anwendung von Koagulationsinstrumenten ist, dass die Koagulationselektroden unter Umständen mit anhaftendem Gewebe verschmutzt werden können. Die anhaftenden Gewebeteile werden dabei ausgetrocknet, wodurch der Stromfluss aufgrund der Erhöhung des Widerstandes zurückgeht.

Um die Anhaftung von Gewebe zu erschweren, ist es bekannt, an Koagulationselektroden Antihaftbeschichtungen anzubringen.

Diese Antihaftbeschichtungen haben allerdings den Nachteil, dass sie das Anhaften von Geweberesten wegen der Erwärmung nicht zuverlässig verhindern können und nach längerem Operationseinsatz nicht mehr gebraucht werden können. Mehrere Reinigungen mit der Drahtbürste, um die stark haftenden Gewebereste zu entfernen, führen zur Zerstörung der Antihaftbeschichtung und des ursprünglich gewünschten Effekts.

US 2006 00 4356 offenbart ein Koagulationsinstrument mit zwei Koagulationselektroden welche jeweils einen Wärmeleitkanal aufweisen.

Die Aufgabe der Erfindung besteht daher darin, ein Koagulationsinstrument bereitzustellen, welche ein Anhaften von Gewebe erschwert.

Die Aufgabe der Erfindung wird gelöst durch ein Koagulationsinstrument mit den Merkmalen des Patentanspruchs 1.

Vorteilhafte Ausgestaltungen und Weiterbildungen der Erfindung sind in den Unteransprüchen angegeben.

Erfindungsgemäß ist in den Koagulationselektroden des Koagulationsinstruments ein Wärmeleitkanal angeordnet. Dieser leitet die Wärme in den Schaftbereich und verhindert auf diese Weise ein Anhaften von Gewebe.

Vorzugsweise ist zur Bildung des Wärmeleitkanals im Kern der Elektroden eine Aussparung angebracht, die mit Silber ausgefüllt ist. Vorteilhafterweise erstreckt sich die Aussparung durch die Arbeitsfläche und den Schaft der Koagulationselektrode, wobei besonders bevorzugt das Volumen der Aussparung im Schaft um ein mehrfaches höher ist als das Volumen der Aussparung in der Arbeitsfläche. Dadurch wird die Wärme mit hoher Geschwindigkeit in den Schaftbereich weitergeleitet mit der Konsequenz, dass ein Verkleben der Elektrode im Arbeitsbereich nicht mehr möglich ist. Als Zusatzeffekt kann die Leistung des HF(Hochfrequenz)-Gerätes auf einen Bruchteil gesenkt werden, da die Silbereinlage die elektrische Leitfähigkeit beispielsweise gegenüber einer Koagulationselektrode aus Stahl um das Mehrfache verstärkt. Insgesamt wird die Anwendung dadurch patientenfreundlicher.

Gemäß einer bevorzugten Ausführungsform der Erfindung sind die Koagulationselektroden bis auf die Arbeitsfläche von einer Beschichtung abgedeckt. Als Beschichtung wird insbesondere Rilsan® verwendet, ein thermoplastischer Kunststoff. Dies ermöglicht eine Manipulation und eine vorsichtige Erweiterung des Operationsfeldes auf sanfte Art. Außerdem kommt es nicht zu unterwünschten Verbrennungen an engen Stellen und in der Tiefe, da es nur an den Arbeitsflächen zur Koagulation kommen kann.

Die Erfindung wird anhand der nachfolgenden Figuren ausführlich erläutert. Es zeigt
- Figur 1: eine schematische Darstellung eines Ausführungsbei- spiels eines Koagulationsinstruments,
- Figur 2: eine Seitenansicht einer der Koagulationselektroden des Koagulationsinstruments gemäß Figur 1 und
- Figur 3: eine Draufsicht auf die Koagulationselektrode gemäß Figur 2.

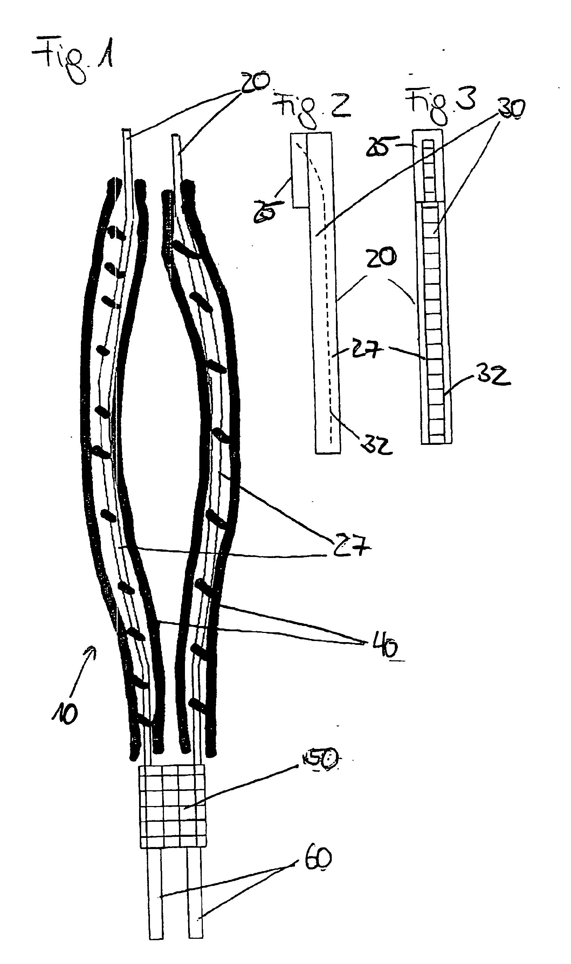

Die Figur 1 zeigt ein Ausführungsbeispiel eines Koagulationsinstruments 10 mit zwei Koagulationselektroden 20, wobei in den Figuren 2 und 3 das distale Ende einer der Koagulationselektroden 20 in einer Seitenansicht und einer Draufsicht vergrößert dargestellt ist.

Das Koagulationsinstrument 10 ist nach Art einer Pinzette betätigbar, wobei die beiden Koagulationselektroden 20 mit ihren distalen Enden gegeneinander bewegt werden. Die Koagulationselektroden 20 weisen jeweils einen Schaft 27 und an ihren freien Enden eine Arbeitsfläche 25 auf. Die Arbeitsflächen 25 sind an den Innenseiten der Koagulationselektroden 20 angeordnet, so dass sie aufeinander zu bewegt werden, wenn die beiden Koagulationselektroden 20 gegeneinander bewegt werden. In der Innenseite der Koagulationselektroden 20 ist eine Aussparung 32 angeordnet, welche sich über die Arbeitsflächen 25 bis in den Schaft 27 hinein erstrecken. Die Aussparung 32 ist mit Silber ausgefüllt und bildet auf diese Weise einen Wärmeleitkanal zur Abfuhr von Wärme aus der Arbeitsfläche 25 in den Schaft 27. Das Volumen der Aussparung des Schafts 27 ist um ein mehrfaches höher als in der Arbeitsfläche 25, um die Wärme mit hoher Geschwindigkeit effektiv in den Schaftbereich abzuleiten.

Die Koagulationselektroden 20 sind abgesehen von ihren Arbeitsflächen 25 mit einer Beschichtung 40 versehen, wobei als Material für die Beschichtung Rilsan® gewählt ist. Die Beschichtung 40 dient insbesondere zur Isolierung der Koagulationselektroden 20.

Die Koagulationselektroden 20 sind im Bereich ihrer proximalen Enden über eine Isolierung 50 miteinander verbunden, wobei die proximalen Enden als Kontakte 60 für einen Stecker dienen.

## Patentansprüche

1. Koagulationsinstrument (10) mit zwei Koagulationselektroden (20), welche jeweils eine'Arbeitsfläche (25) zur Koagulation aufweisen, wobei die Koagulationselektroden (20) einen Wärmeleitkanal (30) aufweisen, wobei
zur Bildung des Wärmeleitkanals (30) im Kern der Koagulationselektroden (20) eine Aussparung (32) angebracht ist, die mit Silber ausgefüllt ist, **dadurch gekennzeichnet dass** sich die Aussparung (32) durch die Arbeitsfläche (25) und den Schaft (27) der Koagulationselektrode (20) erstreckt und dass das Volumen der Aussparung (32) im Schaft,(27) um ein mehrfaches höher ist als das Volumen der Aussparung (32) in der Arbeitsfläche (25).

2. Koagulationsinstrument nach Anspruch 1,
**dadurch gekennzeichnet, dass** die Koagulationselektroden (20) bis auf die Arbeitsfläche (25) von einer Beschichtung (40) abgedeckt sind.

3. Koagulationsinstrument nach Anspruch 2,
**dadurch gekennzeichnet, dass** die Beschichtung (40) aus Rilsan® besteht.

## Claims

1. A coagulation instrument (10) with two coagulation electrodes (20) which in each case have an operative face (25) for coagulation, wherein the coagulation electrodes (20) have a heat-conducting channel (30), wherein a recess (32) which is filled with silver is attached to form the heat-conducting channel (30) in the core of the coagulation electrodes (20), **characterized in that** the recess (32) extends through the operative face (25) and the shaft (27) of the coagulation electrode (20), and the volume of the recess (32) in the shaft (27) is greater by a multiple than the volume of the recess (32) in the operative face (25).

2. A coagulation instrument according to claim 1, **characterized in that** apart from in the operative face (25) the coagulation electrodes (20) are covered by a coating (40).

3. A coagulation instrument according to claim 2, **characterized in that** the coating (40) consists of Rilsan®.

## Revendications

1. Instrument de coagulation (10) comportant deux électrodes de coagulation (20), chacune de ces électrodes comportant une surface de travail (25) pour permettre la coagulation, les électrodes de coagulation (20) comportant un canal de transfert de la chaleur (30), et pour former ce canal de transfert de la chaleur (30), un évidement (32) rempli d'argent étant prévu dans le noyau des électrodes de coagulation (20),
**caractérisé en ce que**
l'évidement (32) s'étend au travers de la surface de travail (25) et de la tige (27) des électrodes de coagulation (20), et
le volume de l'évidement (32) dans la tige (27) est plusieurs fois supérieur au volume de cet évidement (32) dans les surfaces de travail (25).

2. Instrument de coagulation conforme à la revendication 1,
**caractérisé en ce que**
les électrodes de coagulation (20) sont recouvertes d'un revêtement (40) jusqu'aux surfaces de travail (25).

3. Instrument de coagulation conforme à la revendication 2,
**caractérisé en ce que**
le revêtement (40) est réalisé en Rilsan^{®}.
